# EUROPÄISCHE PATENTSCHRIFT

(11) **EP 1 738 159 B1**
(45) Veröffentlichungstag und Bekanntmachung des Hinweises auf die Patenterteilung: **19.08.2009**
(21) Anmeldenummer: 05738163.4
(22) Anmeldetag: 21.04.2005
(51) Int. Cl.: G01N 27/414

(54) **FET-BASIERTER GASSENSOR**
FET-BASED GAS SENSOR
CAPTEUR DE GAZ BAS SUR UN TRANSISTOR À EFFET DE CHAMP

(30) Priorität: 22.04.2004 DE 102004019641
(43) Veröffentlichungstag der Anmeldung: 03.01.2007
(73) Patentinhaber: Micronas GmbH, 79108 Freiburg (DE)
(72) Erfinder: FLEISCHER, Maximilian, 85635Höhenkirchen (DE); MEIXNER, Hans, 85540 Haar (DE); SIMON, Elfriede, 80639 München (DE); LAMPE, Uwe, 21614 Buxtehude (DE); POHLE, Roland, 85570 Herdweg (DE)
(74) Vertreter: Patentanwälte Westphal, Mussgnug & Partner
(86) Internationale Anmeldenummer: PCT/EP2005/004278
(87) Internationale Veröffentlichungsnummer: WO 2005/103666

(56) Entgegenhaltungen:
- DE-A1- 10 121 262
- PATENT ABSTRACTS OF JAPAN Bd. 013, Nr. 263 (P-886), 19. Juni 1989 (1989-06-19) & JP 01 059049 A (DAIKIN IND LTD), 6. März 1989 (1989-03-06)
- PATENT ABSTRACTS OF JAPAN Bd. 015, Nr. 347 (P-1246), 3. September 1991 (1991-09-03) & JP 03 131749 A (SHINDENGEN ELECTRIC MFG CO LTD), 5. Juni 1991 (1991-06-05)

## Beschreibung

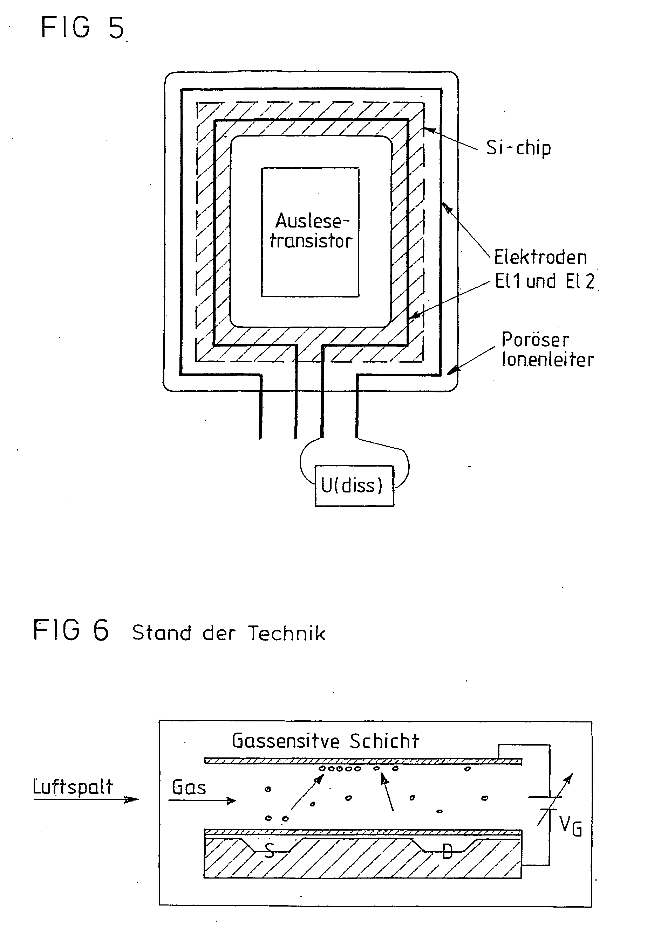

Gassensoren auf Basis von Feldeffekttransistoren messen die Austrittsarbeitsänderung zwischen einem gassensitiven Material und einem Referenzmaterial. Ein typischer Aufbau ist in Figur 6 dargestellt. Der gatelose Transistor ist über einen den Gaseinlass bildenden Luftspalt von wenigen µm Höhe kapazitiv an eine gassensitive Schicht gekoppelt. Bei Kontakt des Gases mit dieser sensitiven Schicht ändert sich das Oberflächenpotential, wodurch der Transistor angesteuert wird. Die Arbeitstemperaturen derartiger Sensoren liegen zwischen Umgebungstemperatur und 150°C.
Eine Ausführung eines derartige Gassensoraufbaues ist ein sogenannter SGFET (suspended gate FET), der z.B. aus der DE 10121262 A1 und der DE 42 39 319 C2 bekannt ist und in der Figur 7 gezeigt wird.

Andere Aufbauten verwenden eine Struktur, bei der der Luftspalt mit gassensitiver Schicht und die FET Struktur zur Auslesung getrennt sind, wie beispielsweise in Figur 8 dargestellt. Derartige Strukturen, genannt CCFET-Strukturen, sind aus DE 4333875 C2 bekannt.

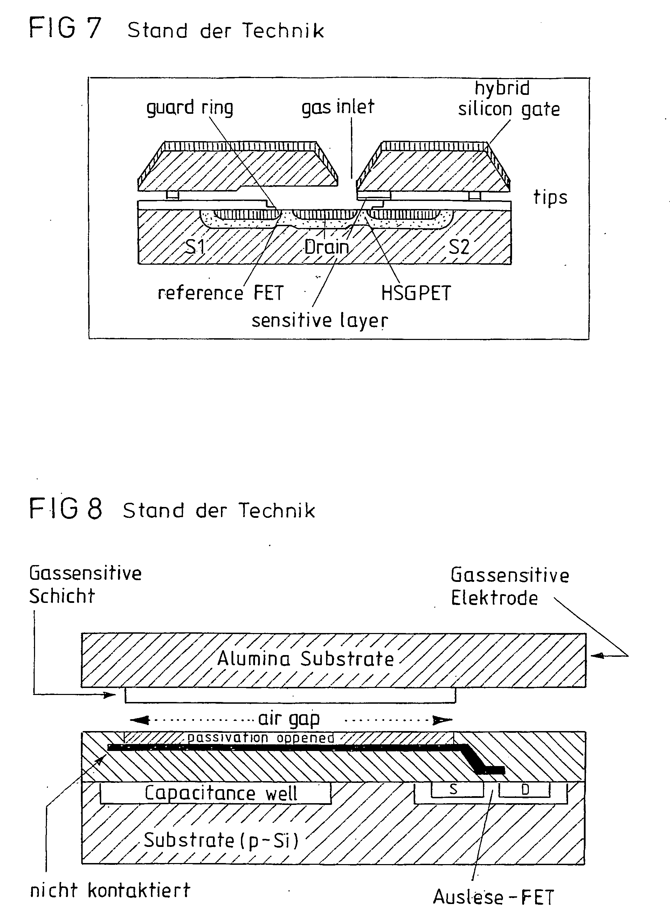

Entsprechend Figur 8 wird der schematische Aufbau eines Gassensors auf Basis des Feldeffekttransistors am Beispiel eines CCFET dargestellt - Stand der Technik. Der Auslese-FET, dargestellt durch die Kontakte Source S und Drain D, wird über ein nicht kontaktiertes Gate, das zusammen mit der gegenüberliegenden gassensitiven Elektrode eine Kondensatoranordnung bildet, angesteuert.

Bei all diesen Gassensoren treten häufig neben Reaktionen auf die zu messenden Zielgase auch unerwünschte Signale von anderen Gasen auf, die u.U. in mehrfacher Intensität dem Nutzsignal überlagert sind und damit zu Fehlmessungen führen. Zusätzlich kann die Signalhöhe auf das Zielgas durch die Anwesenheit eines Störgases verändert werden.

Verbesserungen im Stand der Technik zur Vermeidung von Nachteilen bestanden in:
- einer Optimierung der sensitiven Materialien.
   Durch Optimierung der gassensitiven Materialien und des auf dem Transistor befindlichen Referenzmaterials hinsichtlich der Selektivität kann der Einfluss von Störgasen in gewissem Umfang verringert werden.
- einer Kompensation mittels eines Referenzsensors.
   Ist das Störgas bekannt, kann über einen zweiten für dieses Störgas sensitiven Sensor der Einfluss des Störgases kompensiert werden. Dabei besitzt in Realität dieser zweite Sensor wieder eine nur beschränkte Selektivität, so dass eine zusätzliche Querempfindlichkeiten des Sensorsystem geschaffen wird. Außerdem ist der Referenzbezug wenig erfolgsversprechend, wenn die Höhe des Störsignals die des Nutzsignals um ein mehrfaches übersteigt.
- dem Einsatz von Filtern.
   Störgase lassen sich durch einen für das Zielgas permeablen, für Störgase jedoch undurchlässigen Filter unterdrücken. Dabei wird zum einen das Störgas durch Adsorption z. B. an Aktivkohle entfernt. Hier ist aber im Langzeitbetrieb die begrenzte Filterkapazität zu betrachten, wobei bei Erreichen der Filterkapazität das Gas den Filter durchbrechen kann.
   Bei Sensoren, die bei erhöhten Temperaturen betrieben werden, kann das Störgas über eine chemische Reaktion in Komponenten umgewandelt werden, die kein Sensorsignal hervorrufen, wie z.B. katalytische Filter zur Zersetzung von Alkoholen, bekannt aus DE 43 10 914. Derartiger Filter weisen die o.g. Probleme mit dem Erreichen der Filterkapazität nicht auf. Um derartige Filter benutzen zu können sind jedoch i. A. Temperaturen >300°C nötig, so dass derartige Verfahren für Bauelemente mit Si-Chips und einer maximalen Arbeitstemperatur von ca. 150°C nicht genutzt werden können.

Der Erfindung liegt die Aufgabe zugrunde die Selektivität von FET-basierten Gassensoren zu erhöhen.

Die Lösung geschieht sowohl durch die Merkmalskombination des Anspruches 1, wie auch durch die des Anspruches 10.
Vorteilhafte Ausgestaltungen können den Unteransprüche entnommen werden.

Die Erfindung ergänzt im allgemeinen einen Aufbau eines FET-basierten Gassensors, der FETs nach dem Stand der Technik gemäß der Figur 7 oder Figur 8 durch Weiterbildung mittels eines Kanals, durch den das Gas diffundieren muss, um in den Detektionsbereich des Sensors zu gelangen. In diesem Kanal/Luftspalt befindet sich eine sensitive Schicht, wobei zugleich in diesem Kanal ein elektrochemisches, Element eingebracht ist, welches durch gezielte elektrochemische Umwandlung Störgase zersetzt und zugleich das Zielgas durchlässt.

Dabei gilt, dass der Gaskanal zur Zuführung von Messgas durch eine Verlängerung des Luftspaltes zwischen Kanalisolierung des FETs und der in hybrider Technologie aufgebrachten sensitiven Schicht auf einem Trägersubstrat realisiert ist. Das in den Gaszuführungskanal zum sensitiven Bereich hin eingebrachte elektrochemische Element besteht aus mindestens zwei aktiven Elektroden, an die eine Spannung angelegt ist, wobei mindestens eine in direktem Kontakt mit dem relevanten Messgasgemisch stehen muss, sowie mindestens eine bei typischer Betriebstemperatur des FET mit einem aktiven Ionenleiter kontaktiert ist.
Die Begrenzung der nach innen gerichteten" Gasdiffusion ist so bemessen, dass das elektrochemische Element fähig ist, genügend Störgas in der Applikation zu einem in dem aktiven Sensorbereich nicht mehr detektierbaren Gas umzuwandeln.

Bei Verwendung eines Protonenleiters (Wasserstoff-lonenleiters) können in vergleichbarer Weise sowohl oxidierbare wie auch reduzierbare Störgase abgebaut werden.

Im Folgenden werden anhand der begleitenden die Erfindung nicht einschränkenden Figuren Ausführungsbeispiele beschrieben.
- Figur 1: zeigt den schematischen Grundaufbau der eines Sensors nach der Erfindung mit einem Ionenleiter und aktiven Elektroden El 1 und El2,
- Figur 2: zeigt eine Realisierung des elektrochemischen Filters mit einer Elektrode im Gasdiffusionskanal und einer zweiten Elektrode außerhalb des Gasdiffusionskanals,
- Figur 3: zeigt eine Realisierung des elektrochemischen Filters mit einem Elektrodenpaar im Gasdiffusionskanal auf dem keramischen Trägersubstrat,
- Figur 4: zeigt eine Realisierung des elektrochemischen Filters mit getrennten Elektroden auf dem keramischen Trägersubstrat und dem Sensorchip,
- Figur 5: zeigt eine mögliche Aufsicht für die in Figur 4 vorgeschlagene Variante mit ringförmigen aktiven Elektroden,
- Figur 6: zeigt den schematischen Aufbau eines Gassensors auf Basis des Feldeffekttransistors am Beispiel eines SGFET (suspended gate FET) nach dem Stand der Technik,
- Figur 7: zeigt die bekannte Ausführung eines SGFET,
- Figur 8: zeigt den schematischen Aufbau eines Gassensors auf Basis eines Feldeffekttransistors am Beispiel eines CCFET nach dem Stand der Technik.

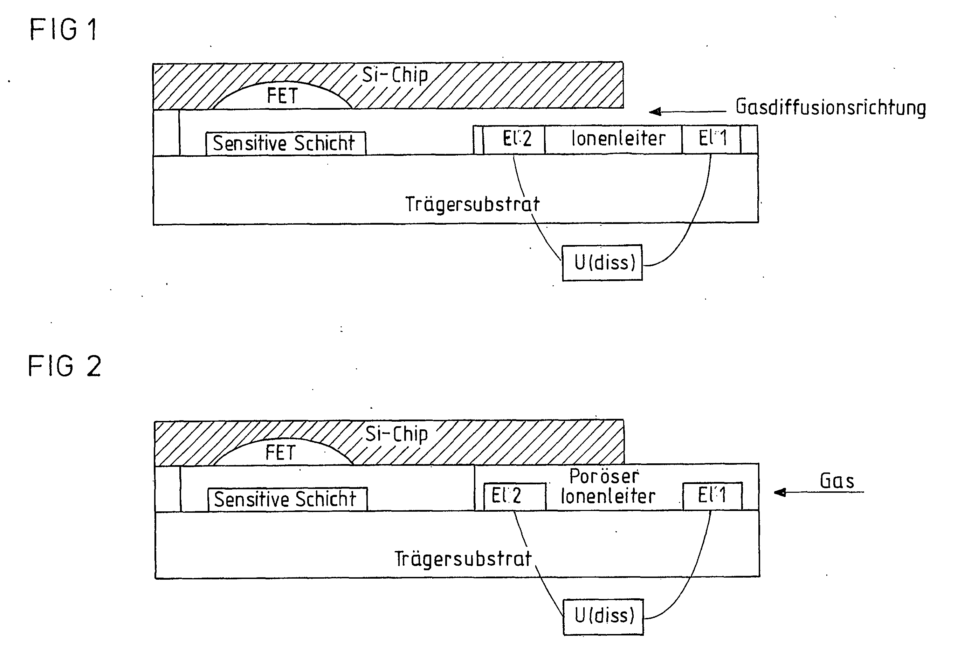

Der Grundaufbau eines erfindungsgemäßen FETs ist in Figur 1 dargestellt. Dieser schematische Grundaufbau eines Gassensors sieht in der Figur rechts neben dem Detektionsbereich mit sensitiver Schicht und Transducer (z.B. CCFET oder SGFET) ein elektrochemisches Element mit dem Ionenleiter und aktiven Elektroden El 1 und El 2 vor.

In dem in Figur 1 gezeigten Aufbau befindet sich die Elektrode El 2 des elektrochemischen Aufbaus im Luftspalt und setzt gezielt Störgase um, die Elektrode El 1 befindet sich außerhalb des Luftspaltes und sorgt mit einer Komplementärreaktion für den Ladungsausgleich im Ionenleiter.

### Materialien für den verwendeten Aufbau:

Das Trägersubstrat ist hierbei üblicherweise ein beliebiger Nichtleiter, der nur die Präparation der sich darauf befindlichen Schichten zulassen muss. Beispiele hierfür sind Keramiken, wie beispielsweise Al₂O₃, ALN oder Si₃N₄, Glas oder auch geeignete Polymermaterialen, z.B. übliche PCB Materialien wie FR4 o.ä..
Die aktiven Elektroden bestehen, zumindest an ihrer Oberfläche aus einem stabilen und katalytisch aktiven Metall. Gut geeignet sind generell Metalle der Platingruppen und ihre Legierungen, z.B. Pt, Pd, PT/Rh oder auch Silber.
Die die beiden Elektroden verbindende Schicht besteht aus einem im Temperaturbereich < 150°C ionenleitendem Material.

Der Gaszutritt bzw. die Diffusion zum Sensorbereich wird durch die Geometrie des Gaseinlasses so stark begrenzt, dass die geringen Gasmengen die vom elektrochemischen Filter umgesetzt und weitergeleitet werden können und eine für die Detektion des Zielgases ausreichende Menge desselben durchlassen.

Ein Betriebsverfahren zur selektiven Detektion eines Zielgases in einem Gasgemisch wird beschrieben, wobei im Sensorbereich, der durch die gassensitive Schicht und den Auslesetransistor umrissen ist, Zielgas detektiert wird, und wobei das zu untersuchende Messgasgemisch vorher durch den oben genannten elektrochemischen Filter so modifiziert wird, dass die selektive Detektion des Zielgases im Sensorbereich möglich ist.

### Grundfunktion des elektrochemischen Filters:

Je nach Polarität der angelegten Gleichspannung U_{diss} erlangt jeweils eine der beiden Elektroden El 1 oder El 2 eine oxidierende Wirkung und die andere eine reduzierende Wirkung auf das im Spalt diffundierende Gas. Wenn z.B. El 2, welche sich entsprechend Figur 1 im Spalt befindet als oxidierende Elektrode gewählt wird, können dadurch Störgase, die leichter oxidierbar sind als das Zielgas, durch Oxidation in Gase umwandelt werden, die keine Querempfindlichkeit mehr verursachen. Wichtig ist hier, das sowohl der Charakter des Verhaltens der Elektrode wie auch Ihre Oxidations- oder Reduktionsstärke durch Wahl der Spannung U_{diss} bestimmt werden kann.

Selbstverständlich können mit dem angegebenen Verfahren nur oxidierbare Störgase entfernt werden, die leichter oxidierbar sind als das Zielgas. Selbiges gilt analog für reduzierbare Gase.

Beispiele üblicher elektrochemisch abzureagierender Gase sind in folgender Liste angeführt:

| Reduktion des Gases durch reduzierend wirkende Elektrode im Gasdiffusionsspalt | |
|---|---|
| Ursprüngliches Gas | Umgewandelt zu |
| NO₂ | NO |
| NO | N² |
| O₃ | O₂ |

| Oxidation des Gases durch oxidierend wirkende Elektrode im Gasdifussionsspalt | |
|---|---|
| HC (Kohlenwasserstoffe) | H₂O und CO₂ |
| Alkohole, Ketone, Aldehyde | H₂O und CO₂ |
| H₂ | H_{2O} |
| NH₃ | N₂ und H_{2O} |
| CO | CO₂ |

Bei einem elektrochemischen Filter kann durch gezieltes Anlegen einer Spannung bestimmter Polarität und Größe die elektrochemische Umsetzung unterschiedlicher Gaskomponenten bewirkt werden.
Je nach Polung der Elektroden kann diese Anordnung entweder zur Abreaktion oxidierender oder zur Abreaktion reduzierender Gase genutzt werden. Die Größe der dabei anzulegenden Spannung liegt hier zwischen typischerweise 200mV und 2V.

Bei Verwendung eines Sauerstoff-Ionenleiters besteht oftmals der Wunsch, mit FET Gassensoren reduzierend wirkende Gase oder auch CO₂ zu detektieren. Hier kann bei manchen Sensorschichten, das in der Anwendung gleichzeitig auftretende oxidierend wirkende NO₂ zu deutlichen Störreaktionen führen. Durch Anlegen einer negativen Spannung and El 2 wird gemäß

NO₂ + 2e⁻ → NO + O²⁻

das reaktive NO₂ zu dem üblicherweise nicht störenden NO umgesetzt. Durch eine stärkere negative Spannung kann auch das NO restlos zersetzt werden:

2 NO + 4e⁻ → N₂ + 2 O²⁻

Das Sauerstoffion wird vom Ionenleiter aufgenommen.

An der Elektrode El 1 findet als Gegenreaktion die Bildung gasförmigen Sauerstoffs statt.

2 O²⁻- 4e⁻ → O₂

Mit umgekehrter Polarität können an El 1 ungewünschte reduzierbare Gase entfernt werden, wie z.B., gemäß

2 NH₃ + 3 O²⁻ → N₂ + 3 H₂O + 6 e⁻

2 H₂ + 2 O²⁻ → 2 H₂O + 4 e⁻ .

An der Elektrode El 1 findet als Gegenreaktion die Aufnahme gasförmigen Sauerstoffs statt.

O₂ + 4e⁻ → 2 O²⁻

Bei Verwendung eines Protonenleiter (Wasserstoffionenleiter):
- Können in vergleichbarer Weise sowohl oxidierbare wie auch reduzierbare Störgase abgebaut werden.
- Der Abbau von NO₂ geschieht in diesem Fall in Anwesenheit von Wasserstoff oder einem anderen wasserstoffhaltigen Gas, wie NH₃, CHₓ, usw. in der Atmosphäre an El 2, z.B. durch

   NO₂ + 2e⁻ +2 H⁺ → NO + H₂O.

An der Elektrode El 1 findet als Gegenreaktion die Aufnahme gasförmigen Wasserstoffs als Wasserstoffion in den Protonenleiter statt.

H₂ → 2 H⁺ + 2e⁻

Obige Reaktion kann natürlich direkt zum Entfernen von Wasserstoff verwendet werden, falls sich die so geschaltete Elektrode im Spalt befindet. Ähnlich kann mit einer oxidierenden Schaltung von El 1 z.B. NH₃ oder Kohlenwasserstoff HC oxidiert werden.

2 NH₃ - N₂ + 6 H⁺ + 6 e⁻

6 H⁺ +6 e⁻ → 3 H₂

Vergleichbares gilt auch für andere Ionenleiter.

Als Materialien für den Ionenleiter werden Materialien bevorzugt, die im Temperaturbereich < 150°C ionenleitende Eigenschaften aufweisen. Beispiele sind im folgenden aufgeführt:
Ein Sauerstoffionenleiter ist beispielsweise LaF3.

### Wasserstoffionenleiter:

z.B. Hydrogen Uranyl Phosphate Tetrahydrate (HUO₂POg*4H₂O), NH₄TaWO₆, NAFION, Aluminiumslikat + Na₂O oder Li₂O oder K₂O, ionenleitende Natrium-Verbindungen wie z.B. Nasicon Na₁₊ₓZr₂ P₃₋ₓSiₓO₁₂, Na₅YSi₄O₁₂;
ionenleitende Lithium-Verbindungen wie Lithium Nitrid, Lithium Titan Phosphat.

### Besondere Vorteile der Erfindung:

Durch die beschriebene Anordnung kann die Selektivität eines Gassensor-Systems wesentlich erhöht werden, indem Gase, die zu Fehlmessungen führen, entfernt werden.

Gegenüber herkömmlichen Filtern besitzen elektrochemische Filter den ausschlaggebenden Vorteil, dass sie nicht verbraucht oder gesättigt werden, dass also langzeitstabiler Dauerbetrieb möglich ist. Zudem sind diese von der Bauform her kleiner.

Elektrochemische Filter sind keine statisch wirkenden Filter, ihre einfache Steuerung durch die Dissoziationsspannung U_{diss} erlaubt die Realisierung von Selbstüberwachungs- und Selbstkalibrierungsfunktionen.

Es besteht bei erfindungsgemäßen Gassensoren der Wunsch, die zuführende Gasdiffusion möglichst klein zu halten, um mit einer möglichst kleinen elektrochemischen Leistung des Filters eine vollständige Entfernung der störenden Gase zu bewirken. Dies realisiert die Ausführungsform gemäß Figur 2. Hier füllt der Ionenleiter den kompletten Gasdiffusionsspalt aus, wodurch es bei vorhandener offenporiger Struktur möglich ist, den Gaszutritt zur sensitiven Schicht zu gewährleisten.

Figur 2 zeigt dien Realisierung des elektrochemischen Filters mit einer Elektrode im Gasdiffusionskanal und einer zweiten Elektrode außerhalb des Diffusionskanals auf dem keramischen Trägersubstrat. Der Gaszutritt erfolgt durch den porös ausgebildeten Ionenleiter.

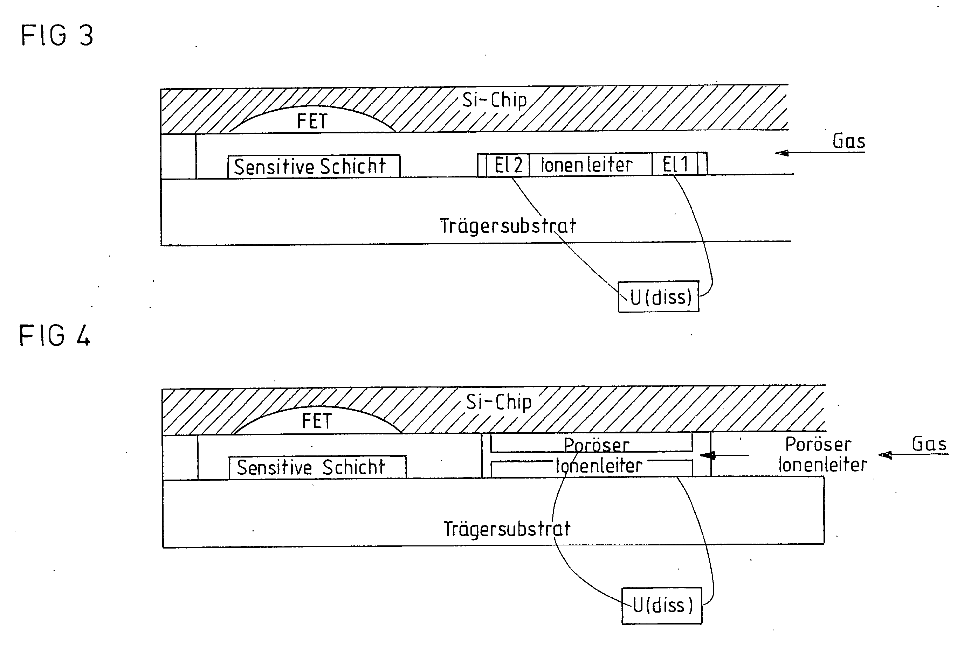

Eine interessante Weiterbildung der Erfindung ist in Figur 3 dargestellt. In dieser Anordnung befinden sich beide Elektroden im engen Gasdiffusionsspalt. Mit einem derartig konfigurierten elektrochemischen Filter werden sowohl reduzierende wie auch oxidierende Gase zersetzt. Die Zersetzungsschwellen lassen sich mittels des angelegten Potentials einstellen. Eine derartige Anordnung ist besonders dazu geeignet, selektiv bezüglich des Redoxverhaltens inerte Gase wie z.B. CO₂ zu detektieren, die durch den Filter nicht verändert werden.

In Figur 4 ist eine Ausführung dargestellt, in der die Charakteristika der Figur 2 und Figur 3 zusammengefasst sind. Beide Elektroden befinden sich im Luftspalt/Messgaskanal, der Gaszutritt erfolgt über den porösen Ionenleiter. Zusätzlich ist hier die Geometrie der Elektroden verändert, indem die gegenüberliegenden Elektroden eine größere Oberfläche haben und damit die Wechselwirkung zwischen Gas und Elektrode verbessert ist.

In Figur 5 ist eine mögliche Ausführung der Elektroden als ringförmige Struktur dargestellt. So kann im Vergleich zu einem einseitig abgeschlossenen Aufbau ein wesentlich größerer Gaseinlass realisiert werden und damit der Gasaustausch zugunsten der Ansprechzeit verbessert werden.

Des weiteren können in erfindungsgemäßen Aufbauten auch mehrstufige elektrochemische Filter vorgesehen werden, d.h. es befinden sich mehrere EL 2 im Gasdiffusionsspalt, die aus unterschiedlichen Materialien bestehen und/oder mit unterschiedlichen Spannungen betrieben werden.

### Selbstüberwachungs- und Selbstkalibrierungsfunktionen:

Die Filterwirkung der elektrochemischen Filter wird durch die elektrische Ansteuerung von El 1 und EL 2 gezielt erzeugt. Durch Variation der Ansteuerspannung U_{diss} kann eine Variation der Filterwirkung erzielt werden, die selbst zur Abreaktion des Zielgases verwendet werden kann.
Dies ermöglicht neben Selbstüberwachungsfunktionen auch eine sequentielle Messung mehrerer Gase, wie z.B. durch kontinuierliche Steigerung der Filterwirkung.
Wird der elektrochemische Filter für das zu detektierende Gas derart ausgelegt, kann durch Modulation der Ansteuerspannung das Zielgas vom Detektionsbereich entfernt werden und so ein künstlicher Nullpunkt geschaffen werden. Somit können Basislinien-Schwankungen des Sensors effizient eliminiert werden.

### Aktivierung des Zielgases

Ein nicht detektierbares Gas kann durch die beschriebene Anordnung in ein Gas umgewandelt werden, auf das das verwendete gassensitive Material reagiert.

## Patentansprüche

1. FET-basierter Gassensor mit einem Gaskanal für die Diffusion eines Messgases zu einer gassensitiven Schicht, die mit einem FET zur Signalauslesung in Wirkverbindung steht, bei dem der Gaskanal durch eine Verlängerung des Luftspaltes zwischen der Kanalisolierung eines FET und der abgehobenen Gate-Elektrode mit der gassensitiven Schicht dargestellt ist, **dadurch gekennzeichnet, dass** innerhalb des Gaskanals zur elektrochemischen Umwandlung von Störgasen und/oder zur Aktivierung eines Zielgases ein elektrochemisches Element zumindest teilweise eingebracht ist.

2. Gassensor nach Anspruch 1, bei dem das elektrochemische Element im Querschnitt des Messgaskanals derart positioniert ist, dass es diesen an keiner Stelle vollständig ausfüllt.

3. Gassensor nach Anspruch 1 oder 2, bei dem das elektrochemische Element den Querschnitt des Messgaskanals vollständig einnimmt und aus porösem Material besteht.

4. Gassensor nach einem der vorhergehenden Ansprüche, bei dem das elektrochemische Element durch einen Ionenleiter und mindestens zwei aktiven Elektroden, an die eine äussere Spannung angelegt ist, dargestellt ist, wobei beide Elektroden voneinander beabstandet mit dem Ionenleiter verbunden sind und mindestens eine der Elektroden direkt in Kontakt mit dem Messgas steht.

5. Gassensor nach Anspruch 4, bei dem eine Elektrode zur Störgasumsetzung im Messgaskanal und die andere Elektrode für eine Komplementärreaktion für den Ladungsausgleich im Ionenleiter ausserhalb des Messgaskanals angeordnet sind.

6. Gassensor nach Anspruch 4 oder 5, bei dem der Ionenleiter aus einem Material besteht, welches bis150 C temperaturstabil ist.

7. Gassensor nach einem der Ansprüche 4 - 6, bei dem die Elektroden zumindest an der Oberfläche aus einem mechanisch stabilen und katalytisch aktiven Metall bestehen.

8. Gassensor nach einem der Ansprüche 4-7, bei dem der Ionenleiter ein Protonenleiter ist.

9. Gassensor nach einem der vorhergehenden Ansprüche, bei dem die elektrochemischen Elemente mehrstufig ausgebildet sind.

10. Betriebsverfahren zur selektiven Detektion eines Zielgases in einem Messgasgemisch mittels eines Feldeffekttransistors mit an einem abgehobenen Gate integrierter gassensitiver Schicht, **dadurch gekennzeichnet, dass** das zu untersuchende Messgasgemisch vorher durch ein elektrochemisches Element derart aufbereitet wird, dass im Sensorbereich das Messgas neben dem Zielgas minimal Störgasreaktionen verursachende Gase enthält und/oder mindestens ein Zielgas derart aktiviert wird, dass es von einem gassensitiven Material detektiert werden kann.

11. Betriebsverfahren nach Anspruch 11, bei dem an die Elektroden eine äußere Spannung angelegt wird und eine der Elektroden eine oxidierende und die andere eine reduzierende Wirkung aufweist, wobei die daraus ausgewählte Elektrode zur Eliminierung von entsprechenden Störgasen im Messgaskanal positioniert sein muss.

12. Betriebsverfahren nach Anspruch 11 oder 12, bei dem das Potential der Äußeren Spannung variiert wird, um die Dissoziation bzw. Zersetzungsschwellen an bestimmte Gase anzupassen.

13. Betriebsverfahren nach einem der Ansprüche 11 - 13, bei dem zur Selbstüberwachung oder Selbstkalibrierung des Sensors die Filterwirkung der elektrochemischen Filter durch die elektrische Ansteuerung von E1 1 und EL 2 gezielt gesteuert wird, wobei im Extremfall das Zielgas elektrochemisch umgewandelt wird.

## Claims

1. FET-based gas sensor with a gas channel for the diffusion of a measurement gas to a gas-sensitive layer which is in active connection with an FET for reading out a signal, in which the gas channel is created by lengthening the air gap between the channel insulation of an FET and the raised gate electrode with the gas-sensitive layer,
**characterised in that**
within the gas channel, an electrochemical element is at least partially introduced, for the electrochemical conversion of interfering gases and/or to activate a target gas.

2. Gas sensor according to claim 1, in which the electrochemical element is positioned in the cross-section of the measurement gas channel in such a way that it does not completely fill it at any point.

3. Gas sensor according to claim 1 or 2, in which the electrochemical element completely fills the cross-section of the measurement gas channel and consists of porous material.

4. Gas sensor according to one of the preceding claims, in which the electrochemical element is formed by an ion conductor and at least two active electrodes to which an external voltage is applied, wherein the two electrodes are connected to the ion conductor, spaced apart from one another, and at least one of the electrodes is in direct contact with the measurement gas.

5. Gas sensor according to claim 4, in which one electrode is positioned in the measurement gas channel to convert interfering gas, and the other electrode is positioned outside the measurement gas channel, for a complementary reaction for charge compensation.

6. Gas sensor according to claim 4 or 5, in which the ion conductor consists of a material that is stable to temperatures up to 150°C.

7. Gas sensor according to one of the claims 4-6, in which the electrodes consists of a mechanically stable and catalytically active metal, at least on the surface.

8. Gas sensor according to one of the claims 4-7, in which the ion conductor is a proton conductor.

9. Gas sensor according to one of the preceding claims, in which the electrochemical elements are of a multi-stage design.

10. Operating method for the selective detection of a target gas in a measurement gas mixture by means of a field effect transistor with a gas-sensitive layer integrated on a raised gate,
**characterised in that**
the measurement gas mixture that is to be examined is previously prepared by an electrochemical element in such a way that in the sensor area, in addition to the target gas, the measurement gas contains minimal amounts of gases that cause interfering gas reactions, and/or at least once target gas is activated such that it can be detected by a gas-sensitive material.

11. Operating method according to claim 10, in which an external voltage is applied to the electrodes, and one of the electrodes has an oxidising effect and the other one has a reducing effect, wherein the electrode chosen from them for eliminating corresponding interfering gases must be positioned in the measurement gas channel.

12. Operating method according to claim 10 or 11, in which the potential of the external voltage is varied to match the dissociation or decomposition thresholds for particular gases.

13. Operating method according to one of the claims 10-12, in which for the self monitoring or self-calibration of the sensor, the filtering action of the electrochemical filter is selectively controlled by the electrical triggering of El 1 and EL 2, with the target gas being electrochemically converted in extreme cases.

## Revendications

1. Capteur de gaz à transistor FET comportant un canal de gaz pour la diffusion d'un gaz de mesure vers une couche sensible au gaz, coopérant avec un transistor FET pour exploiter un signal, selon lequel le canal de gaz correspond à un prolongement de l'intervalle d'air entre l'isolation de canal d'un transistor FET et l'électrode de porte, relevée, avec la couche sensible au gaz,
**caractérisé en ce qu'**
on a introduit au moins partiellement un élément électrochimique à l'intérieur du canal de gaz, pour la conversion électrochimique de gaz perturbateur et/ou pour activer un gaz cible.

2. Capteur de gaz selon la revendication 1,
selon lequel
l'élément électrochimique est positionné par rapport à la section du canal de mesure de façon à ne pas remplir complètement ce canal.

3. Capteur de gaz selon la revendication 1 ou 2,
selon lequel
l'élément électrochimique occupe complètement la section du canal de mesure et se compose d'un matériau poreux.

4. Capteur de gaz selon les revendications précédentes,
selon lequel
l'élément électrochimique est constitué par un conducteur d'ions et d'au moins deux électrodes actives auxquelles on applique une tension externe,
les deux électrodes écartées l'une de l'autre sont reliées par le conducteur ionique et au moins l'une des électrodes est en contact direct avec le gaz de mesure.

5. Capteur de gaz selon la revendication 4,
selon lequel
une électrode de conversion de gaz perturbateur est installée dans le canal de mesure et l'autre électrode pour une réaction complémentaire pour la compensation de la charge est prévue dans le conducteur ionique en-dehors du canal de mesure.

6. Capteur de gaz selon la revendication 4 ou 5,
**caractérisé en ce que**
le conducteur d'ions est en une matière stable à la température jusqu'à 150°C.

7. Capteur de gaz selon les revendications 4 à 6,
selon lequel
au moins la surface des électrodes est en un métal métallique non stable et actif du point de vue catalytique.

8. Capteur de gaz selon les revendications 4 à 7,
selon lequel
le conducteur d'ions est un conducteur de protons.

9. Capteur de gaz selon les revendications précédentes,
selon lequel
les éléments électrochimiques sont réalisés en plusieurs étapes.

10. Procédé de fonctionnement pour la détection sélective d'un gaz cible dans un mélange de gaz de mesure à l'aide d'un transistor à effet de champ, d'une couche sensible aux gaz, intégrée dans la porte relevée,
**caractérisé en ce que**
le mélange de gaz à examiner est préparé au préalable par un élément électrochimique de façon que dans la plage du capteur, le gaz de mesure contient non seulement à côté du gaz cible, un minimum de gaz occasionnant des réactions perturbatrices et/ou au moins un gaz cible est activé de façon à être détecté par le matériau sensible au gaz.

11. Procédé de gestion selon la revendication 10,
selon lequel
on applique une tension extérieure aux électrodes et l'une des électrodes a un effet oxydant et l'autre un effet réducteur, l'électrode sélectionnée par celle-ci étant positionnée dans le canal de mesure pour éliminer complètement les gaz perturbateurs correspondants.

12. Procédé de gestion selon la revendication 10 ou 11,
selon lequel
on fait varier le potentiel de la tension extérieure pour adapter la dissociabilité ou le seuil de perturbation à des gaz déterminés.

13. Procédé de gestion selon les revendications 10 à 12,
**caractérisé en ce que**
pour la surveillance autonome ou l'autocalibrage du capteur, on commande de manière ciblée l'effet de filtrage du filtre électrochimique par la disposition électrique de E1 et E2, et dans le cas extrême, le gaz cibles est converti par voie électrochimique.
